(19) **Europäisches Patentamt / European Patent Office / Office européen des brevets**

(11) **EP 4 143 337 B1**

(12) **EUROPEAN PATENT SPECIFICATION**

(45) Date of publication and mention
of the grant of the patent:
**10.06.2026 Bulletin 2026/24**

(21) Application number: **21723158.8**

(22) Date of filing: **28.04.2021**

(51) International Patent Classification (IPC):
**C12Q 1/686** (2018.01)    **G16B 25/20** (2019.01)
**B01L 9/00** (2006.01)    **C12Q 1/6851** (2018.01)

(52) Cooperative Patent Classification (CPC):
(C-Sets available)
**G16B 40/10; C12Q 1/6851; C12Q 1/686;**
**G16B 25/20**                                    (Cont.)

(86) International application number:
**PCT/EP2021/061040**

(87) International publication number:
**WO 2021/219675 (04.11.2021 Gazette 2021/44)**

(54) **A METHOD FOR DETECTING REACTION VOLUME DEVIATIONS IN A DIGITAL POLYMERASE CHAIN REACTION**

VERFAHREN ZUR DETEKTION VON REAKTIONSVOLUMENABWEICHUNGEN IN EINER DIGITALEN POLYMERASEKETTENREAKTION

PROCÉDÉ DE DÉTECTION D'ÉCARTS DE VOLUME DE RÉACTION DANS UNE RÉACTION EN CHAÎNE PAR POLYMÉRASE NUMÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2020 US 202063018183 P**

(43) Date of publication of application:
**08.03.2023 Bulletin 2023/10**

(73) Proprietors:
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
**68305 Mannheim (DE)**
Designated Contracting States:
**DE**

(72) Inventors:
• **CHUANG, Han-Yu**
**Pleasanton, California 94588 (US)**

• **HOLMSTROM, Mark Alan**
**Pleasanton, California 94588 (US)**
• **KAMNEVA, Olga Konstantinovna**
**Pleasanton, California 94588 (US)**

(74) Representative: **Merkel, Patrick**
**Roche Diagnostics GmbH**
**Sandhofer Strasse 116**
**68305 Mannheim (DE)**

(56) References cited:
| | |
|---|---|
| WO-A1-2020/219549 | WO-A1-2020/219549 |
| CN-A- 108 444 954 | CN-A- 108 444 954 |
| US-A1- 2016 083 787 | US-A1- 2016 083 787 |
| US-A1- 2018 230 515 | US-A1- 2018 230 515 |

• "Handbook of Image and Video Processing", 14 June 2000, ACADEMIC PRESS, article PETROS MARAGOS ET AL: "Morphological Filtering for Image Enhancement and Detection", pages: 101 - 116, XP055307217, DOI: 10.1016/ B978-012119792-6/50072-3

EP 4 143 337 B1

- "HANDBOOK OF IMAGE AND VIDEO PROCESSING (Second Edition)", 1 January 2005, ELSEVIER ACADEMIC PRESS, Amsterdam, NL, ISBN: 978-0-12-119792-6, article GONZALO R ARCE ET AL: "Nonlinear Filtering for Image Analysis and Enhancement", XP055705971, DOI: 10.1016/B978-012119792-6/50071-1
- "Handbook of Image and Video Processing", 14 June 2000, ACADEMIC PRESS, article PETROS MARAGOS ET AL: "Morphological Filtering for Image Enhancement and Detection", pages: 101 - 116, XP055307217, DOI: 10.1016/B978-012119792-6/50072-3
- "HANDBOOK OF IMAGE AND VIDEO PROCESSING (Second Edition)", 1 January 2005, ELSEVIER ACADEMIC PRESS, Amsterdam, NL, ISBN: 978-0-12-119792-6, article GONZALO R ARCE ET AL: "Nonlinear Filtering for Image Analysis and Enhancement", XP055705971, DOI: 10.1016/B978-012119792-6/50071-1

(52) Cooperative Patent Classification (CPC): (Cont.)

C-Sets
**C12Q 1/6851, C12Q 2531/113, C12Q 2537/165; C12Q 1/686, C12Q 2537/165, C12Q 2545/114**

**Description**

**Field of the Disclosure**

The present disclosure relates to a method for detecting reaction volume deviations in a digital polymerase chain reaction (dPCR) and to a dPCR method for determining the amount or concentration of a nucleic acid of interest in a sample that accounts for reaction volume deviations.

**Background**

For many biological, biochemical, diagnostic or therapeutic purposes, it is necessary to accurately and precisely determine the amount or concentration of a nucleic acid in a sample. Digital PCR offers an alternative method to conventional real-time quantitative PCR for absolute quantification of nucleic acids and rare allele detection. Digital PCR works by partitioning a sample of nucleic acids into many individual, parallel PCR reactions; some of these reactions contain the target molecule (positive) while others do not (negative). Following PCR analysis, the fraction of negative reactions is used to generate an absolute count of the number of target molecules in the sample. One of the key advantages of dPCR over real-time PCR is its superior accuracy of quantification. This advantage relies on inherent properties of dPCR as quantification only requires correct counting of positive partitions (or reaction volumes) and the knowledge of the theoretical partition volume (the count number is not very sensitive to PCR efficiency). A quantification standard is not required. This eliminates potential quantification errors caused by the standard itself.

The prior art provides methods to identify incorrect positive or negative counts and for calibrating or normalizing signals in droplet-based assay (US 2013/0302792 A1). This normalization should improve the separation between positive and negative counts. Hence, the normalization reduces the risk of false positive or negative counts. The ultimate goal is to improve the accuracy and precision of the determination of the nucleic acid concentration by correcting the signal obtained for the nucleic acid.

However, prior art methods do not account for errors in PCR due to situations in which the true volume in the dPCR partitions differs from the expected or intended one.

Accordingly, there is a need for methods of quantifying a nucleic acid of interest by dPCR, which accounts for reaction volume deviations. US 2018/230515 discloses a method for reducing quantification errors caused by an optical artefact in dPCR. The filling of a partition can be found inappropriate by using threshold values for the signal.

Summary

The present disclosure provides a method, as defined in claim 1, for detecting reaction volume deviations in a dPCR assay, wherein the dPCR assay is used to quantify an amount or concentration of nucleic acid of interest in an array of partitions. The method includes the following steps:

(a) combining optical signals across (x, y) coordinates within the array using a convolution with a kernel function, wherein each partition is assigned a convolution value; and
(b) identifying valid partitions and void partitions by comparing the convolution value of each partition to a threshold convolution value.

In addition, the method may also include (c) subjecting data collected in step (b) to one or more additional steps comprising: clustering and morphological image processing operations. Such morphological image processing operations can include dilation and/or erosion, whereas clustering can include valid and/or void trimming.

Outside the scope of the claims, the present disclosure also contemplates a method for determining the amount or concentration of a nucleic acid of interest in a sample, the method comprising the steps of: (a) providing a sample suspected of containing the nucleic acid of interest; (b) performing a dPCR with the sample in a dPCR plate comprising an array of partitions; (c) identifying one or more valid partitions in the array of partitions; and (d) calculating the amount or concentration of the nucleic acid of interest as number of nucleic acid as determined in step (b) per valid partition volume. Moreover, the method may also include determining a copy number, $N_c$, of nucleic acid of interest in the one or more valid partitions identified in step (c) and dividing $N_c$ by the valid partition volume.

In a particular embodiment, the disclosure provides a a computer program product comprising instructions to cause a laboratory instrument to execute the steps of the method.

**Brief Description of the Figures**

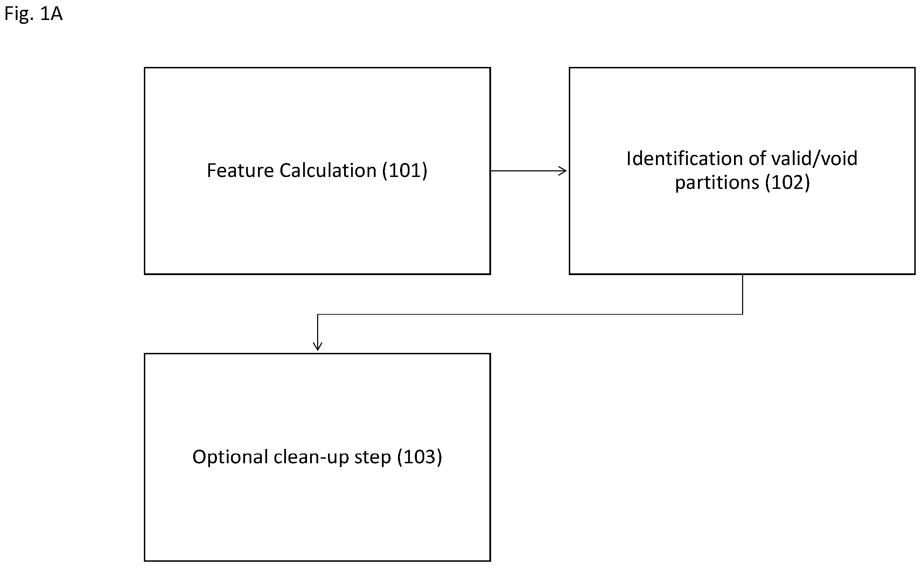

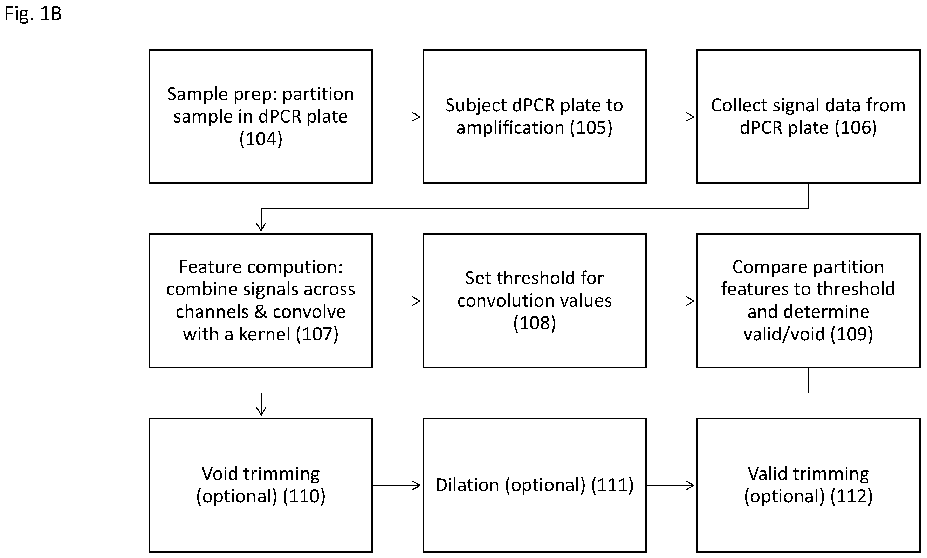

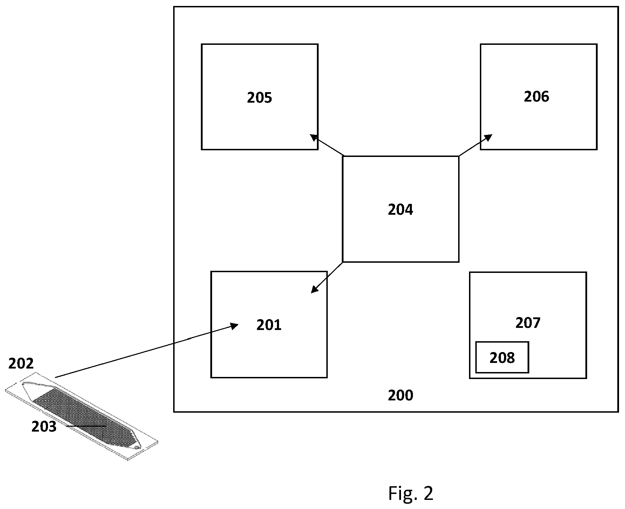

Figs. 1A-1B schematically illustrate the methods described herein for detecting reaction volume deviations in a dPCR assay. Fig. 1A illustrates the method used to analyze a dPCR plate following nucleic acid amplification, and Fig. 1B illustrates the complete method from preparation of the dPCR plate to analysis using the methods described herein. Fig. 2 is a schematic illustration of a laboratory instrument as described herein.

## Detailed Description

As detailed above, a method to reliably determine the amount or concentration of a nucleic acid is of particular relevance in several industrial applications, e.g. in the medical field. Such applications may require a precise and accurate determination of the amount or concentration of the nucleic acid in the sample, e.g. a sample obtained from a patient or product. This might be of interest, e.g., in the diagnosis of the severity of a disease, in environmental technology, or as a means of determining the quality of a product, e.g., in order to define contaminations or impurities.

Digital PCR is a biotechnology refinement of conventional polymerase chain reaction methods that can be used to directly quantify and optionally clonally amplify nucleic acids including DNA, cDNA, RNA or mixtures thereof. The key difference between dPCR and traditional PCR (e.g. qPCR) lies in the method of measuring nucleic acids amount, with the former being a more precise and accurate method than PCR, though also more prone to error in the hands of inexperienced users. The smaller dynamic range of dPCR may require dilutions of the sample. dPCR also carries out a single reaction within a sample, however the sample is separated into a large number of partitions or partitions and the reaction is carried out in each partition or partition individually. This separation enables a more reliable collection and sensitive measurement of nucleic acid amounts. Moreover, the method allows for accurate quantification.

A detailed description of dPCR devices and methods can be found, e.g., in U.S Patent Application No. 20080160525; Vogelstein, et al., Proc. Natl. Acad. Sci. USA, Vol. 96, 9236-9241, Aug. 1999; McCaughan, et al., J. Pathol. 2010; 220: 297-306; Mao, et al., Am. J. Transl. Res. 2019; 11(12): 7209-7222; US Patent No. 10,564,102; US Patent Publication No. 20180045641A1; European Patent No. 3299471B1; US Patent Publication No. 20180147574A1; and US Patent Publication No. 20180087090A1.

In one specific embodiment, the dPCR sample is separated or partitioned in an array comprising a plurality of partitions (also referred to alternatively as reaction volumes or wells) so that individual nucleic acid molecules within the sample are localized and concentrated within many separate regions within the array. The partitioning of the sample allows one to estimate the number of nucleic acids by assuming that the target molecule counts within each partition follow one consistent Poisson distribution. After PCR amplification, each partition is identified as a negative or positive reaction ("0" or "1 or more" molecules, respectively). The target molecules may be quantified by counting the number of positive and negative partitions and then estimating the underlying Poisson distribution using a maximum likelihood estimate. In conventional quantitative PCR, the quantitation result may depend on the amplification efficiency of the PCR process. dPCR, however, is not dependent on the number of amplification cycles to determine the initial sample amount, eliminating the reliance on uncertain exponential data to quantify target nucleic acids and therefore provides absolute quantification.

As the sample is partitioned in the array, the partitioning process may lead to partially filled or unfilled partitions, i.e., a reaction volume deviation, and the partially filled or unfilled partitions are alternatively referred to as voids or fill-voids. The fluorescent signal from a void may be difficult to distinguish from the signal observed from an otherwise filled partition containing low or no target molecule. In addition, bright spots may be observed in void partitions, which may be mistaken for positive signal from filled partitions.

These issues can be addressed using the methods of the present disclosure, illustrated schematically in Fig. 1A. Briefly, voids can be identified and distinguished in the signal data by analyzing regions of the dPCR plate where the signal is consistently low across all channels. The analysis of these low signal regions includes:

a) feature calculation, 101, by combining the optical signals across (x,y) coordinates within the array using a convolution with a kernel function to assign each partition a convolution value;
b) identification of valid or void partitions, 102, by comparing the convolution value of each partition to a threshold convolution value;
c) optionally an additional clean-up step, 103, may be performed that includes subjecting the data collected in step (b) to one or more additional steps including clustering and morphological image processing operations.

Each step of the method is described in more detail below and illustrated in Fig. 1B, with specific reference to an exemplary dPCR plate.

Briefly, in a specific embodiment, a dPCR plate may include 1-8 reaction mixes (samples) within the format of a standard microwell plate (SBS format, not shown in the figures). Each of the 1-8 sample positions within the dPCR plate may consist of an inlet port, e.g., at position A1 of the plate, the microstructured part between positions A1 and A12, and an outlet port at position A12. Once a volume of fluid sample is added to each of the inlet ports, a partitioning fluid is added to each inlet. This can be done manually, using a single or an 8-channel pipette, or by using an automated dispense station.

The separation or partitioning fluid is a hydrophobic liquid that is immiscible and unreactive with respect to the reaction mix, e.g. a long-chain fluorinated hydrocarbon or a silicon oil. The separation of the individual partitions containing reaction mix (or partitioning) can be done passively, or by applying overpressure at the inlet ports, or by applying underpressure at the outlet ports. Monitoring sensors may be used to insure that the process stops when the separation is complete, i.e., the separation fluid has reached the outlet port. The sample preparation step is shown in Fig. 1B, 104.

After partitioning, the dPCR plate is subjected to a thermal cycling process, 105, followed by image analysis to detect fluorescent signals associated with each individual partition in the array. Signal data from the preliminary image analysis step are collected, 106. Table 1 summarizes the data input used in the method described herein:

Table 1

| Name | Level | Description |
|---|---|---|
| XYCoordinates | Lane | Array of X and Y coordinates per partition (partition order equal to Fluorescence) |
| Fluorescence | Channel | Array of fluorescence intensity values per partition (partition order equal to PartitionFlags) |
| PartitionInputFlags | Lane | The partition input flags (partition order equal to Fluorescence) |
| maxChannel | Channel | Max signal per channel as identified by Outlier Removal algorithm |
| useChannel | Channel (conditional) | If the channel is to be used for void determination |
| Radius | Global | Radius to be used during the convolution |
| standard Deviation | Global | SD (or equivalent) to be used in kernel |
| diffOff | Global | How far below the mean void threshold is set |
| voidNoise | Global | Minimal size of void cluster to not be considered noise |
| goodNoise | Global | Minimal size of good cluster to not be considered noise |
| highConvolution Threshold | Global | Minimal convolution mean needed to consider the lane mostly positive |
| lowVarianceThreshold | Global | Maximal MAD needed to be considered low variance |
| thresholdAdjustmentFrac | Global | Fraction of the mean to be used as the alternate threshold |
| cleanupRadius | Global | Radius used in the dilation cleanup step |

The first step in detecting reaction volume deviations is feature computation, 107. The goal of feature computation is to combine the signals in a way that makes the separation between void and valid partition more apparent. Signals are first combined across channels and then convolved with a kernel based on (x,y) coordinates. The signals are convolved with a kernel to refine or "smooth out" the image. In one embodiment, the channels can be convolved individually or the sum of the normalized signals across all of the channels can be convolved.

In a specific embodiment, the signals are summed in order to highlight when a partition is dim in all channels rather than in just one. In order to insure that all partitions contribute equally, the signal values are normalized within each channel. In one embodiment, only those channels with a substantial portion of positives signals are used in the calculation. Channels are selected for use by the useChannel flag, i.e., $Flag_{partition}$ = 1 (Valid). The sum of the signals is calculated as follows:

$$signalChannel = Fluorescence(Channel)_{Partition}$$

$$signalSum[i] = \sum_{useChannel[ch]==TRUE} \frac{singalChannelch[i]}{maxChannelch}$$

A convolution is used to differentiate between dim regions and dim partitions. Convolution includes several steps: first, distance and kernal functions are established. The standard L2 distance and a custom exponential kernel can be used:

$$Dist(x_1, y_1, x_2, y_2) = \sqrt{(x_1 - x_2)^2 + (y_1 - y_2)^2}$$

$$ker(d) = \begin{cases} 2 & d = 0 \\ e^{-1 \times \sigma \times d} & otherwise \end{cases}$$

For the sake of notation, z represents the (x,y) coordinate of a given partition and the convolution of z is represented by:

$$Conv[z] = \frac{\sum_{isValidPartition(z') \wedge Dist(z,z') \leq radius} signalSum[z'] \times ker(Dist(z, z'))}{\sum_{isValidPartition(z') \wedge Dist(z,z') \leq radius} ker(Dist(z, z'))}$$

Note that in the case where *{isValidPartition(z') ^ Dist(z, z') <= radius}* is the empty set, this value is not well defined. In that case, the result is set to an impossible default value, e.g., -1. In general, the focus of the method is on the convolution for the partitions which are valid so the use of an invalid default value such as this is acceptable.

In order to initially differentiate void and valid partitions, a threshold value is set for the convolution values, 108. Any partition with a convolution value of less than the threshold is marked as void and any partition having a convolution value that exceeds the threshold remains valid. The method used to determine the threshold involves analyzing candidate reference regions in the dPCR plate to choose one that represents the filled portion of the plate and then setting the threshold based on the convolution value in that region.

There are many potential reference regions that can be drawn on a dPCR plate. Analyzing every potential reference region would be slow, so a subset is selected, in this example, 6 reference regions. The ideal number of candidate reference regions may vary depending on the size and partition density of the plate. Six regions are used in a representative dPCR plate for illustrative purposes: *(maxx* and *maxy* are the max x and y coordinates of the partitions) For

$$0 \leq i < 4$$

$$ref_i = \left\{ z \mid \left( \frac{i}{5} \times maxy \leq z_y < \frac{i+1}{5} \times maxy \right) \right\}.$$

Note that for the case of *i* = 0 the lower boundary on *y* is moved from 0 to 11 to avoid using a region positioned at the inlet of the plate. The final two reference regions are horizontal instead of vertical:
For

$$j = 0, 1$$

$$ref_j = \left\{ z \mid \left( 11 \leq z_y < 0.5 \times maxy \wedge \frac{i}{3} \times maxx + \frac{maxx}{6} \leq z_x \leq \frac{i}{3} \times maxx + \frac{maxx}{2} \right) \right\}$$

For each reference region, the mean of the valid convolution is calculated (value not equal to the default -1) for that region. The region with the second highest mean convolution is taken as the reference. In this specific embodiment, the second highest is selected instead of the first highest due to the fact that certain image artifacts may amplify the convolution in some areas. The second brightest region is less likely to have these issues but it is very likely to include completely filled partitions.

In order to set the threshold, the expected deviation for the convolution of filled partitions is determined. First, median absolute deviation (MAD) is used:

$$MAD(ref) = median(|mean(ref) - ref[z]|)$$

The default convolution values are excluded from this expression, yielding the following standard threshold:

$$voidThresh = mean(ref) - diffOff \times MAD(ref)$$

Next, the need for an alternative threshold is evaluated. The standard threshold may not be suitable when MAD is very small, e.g., when lambda is very high or very low. While the possible remedies available when lambda is very low are limited, a simple solution can be used to recover a proper threshold when lambda is very high. In this instance, if *mean(ref) > highConvolutionThreshold and MAD(ref) < lowVarianceThreshold,* then the alternate threshold is used.

$$voidThresh = mean(ref) * thresholdAdjustmentFrac$$

With the threshold set, a partition is classified as void if the convolution value is less than the threshold and if the convolution value meets or exceeds the threshold, the partition is deemed valid, 109.

It may be desirable to employ a final cleanup step if the signal is noisy and/or otherwise valid partitions are dim or void partitions contain substantial signal. The cleanup step can include one or more of the following steps: void trimming, 110, dilation, 111, and valid trimming, 112.

(i) Void trimming

Voids can be trimmed by clustering using path-connectedness. Briefly, a given partition's neighbors include those partitions that share a wall with that partition. For example, if partitions in the device are square, rectangular, or hexagonal in shape, then a given partition's neighbors are the 4 or 6 partitions, respectively, that share a wall with that partition. In this example, a path can be made that connects one partition to the next, by starting at one partition and moving to one of its neighbors and then moving to one of that new partition's neighbors and so on. Two void partitions are path-connected if there exists a path between the two partitions that only passes through void partitions. Void partitions can be clustered by path-connectedness by making groups of partitions that are all path-connected to each other. The clusters that are too small, having size less than *voidNoise,* are reclassified as Valid.

(ii) Dilation

After erroneous voids are removed, the remaining voids are dilated to ensure that any boundary voids are removed. Dilation can be done using any suitable brush, e.g., a "diamond-shape" brush with the radius cleanupRadius, i.e., for any valid partition with coordinates z, the partition is reclassified as void if and only if there exists a void partition *z'* with $|z_x - z'_x| + [z_y - z'_y] \leq cleanupRadius$.

(iii) Valid trimming

In the final step, valid partitions are trimmed by removing small groups. This step is performed as described herein for void trimming but for the valid partitions. First, the valid partitions are clustered by path-connectedness and then the clusters with a size less than *goodNoise* are reclassified as Void.

Once voids were properly identified, the flags for those partitions are changed to void.

The output of the algorithm is summarized in Table 2:

Table 2.

| Name | Level | Description |
|---|---|---|
| signalSum | Lane | The scaled signal sum per partition |
| convolution | Lane | The convolution result per partition |
| refMean | Lane | The convolution reference mean |
| voidThresh | Lane | The convolution threshold |
| PartitionOutputFlags | Lane | This parameter is a summary of the PartitionOutputFlagsChannel. If a partition is invalidated on at least one channel level, this output (on lane level) will reflect the invalid state. Select from one of the following:<br><br>-Valid<br><br>-Invalid Void<br><br>-Invalid Other |
| voidPartitionCount | Lane | Count of partitions determined to be void |
| voidParitionFrac | Lane | Fraction of void partitions over all valid partitions |

Once void partitions are identified using the methods described herein, the dPCR system can quantify the amount or concentration of nucleic acid of interest in the valid partitions in the array, disregarding signal data collected from void partitions. In a specific embodiment, the concentration of the nucleic acid of interest in one or more valid partitions is calculated as the number of nucleic acid molecules per valid partition volume.

Accordingly, the concentration may be calculated by dividing the target molecules count (also known as copy number) $N_c$ by the sampled liquid volume. The copy number $N_c$ is derived as follows: first the dPCR system identifies which valid partitions are positive and negative for the target molecule and derives totals for each.

As an example, in a dPCR plate with 2000 negative and 8000 positive valid partitions, then a maximum likelihood estimate is used to calculate the underlying parameter in the Poisson distribution, $\lambda$. An estimate of the probability of the partition being negative can be calculated as follows:

$$P(negative) = \#negatives/\#total.$$

For example, in the exemplary dPCR plate described above:

$$P(negative) = 2000/(2000 + 8000) = 0.2.$$

If X is the Poisson random variable modeling the number of molecules in a partition, then

$$P(negative) = P(X=0) = exp(-\lambda)$$

is used to estimate $\lambda$, the only parameter in the Poisson distribution. Applying this rationale to the exemplary dPCR plate:

$$exp(-\lambda) = P(negative) = 0.2, i.e., \lambda=-log(0.2)=1.61 \text{ (rounded)}.$$

$\lambda$ is an important value since it is also the expected or mean value of the Poisson distribution. In other words, $\lambda$ is the average number of target molecules per partition based on the Poisson estimate. The $\lambda$ estimate is used to determine $N_c$:

$$N_c = \lambda * \#of \text{ filled partitions.}$$

The number of filled partitions is the number of non-void partitions: valid + invalid not void. For the exemplary dPCR plate, assuming 1000 additional partitions which are invalid and not void, then the #of filled partitions = 2000 + 8000 + 1000 = 11000 and the copy number is $N_c$= 1.61 * 11000 = 17710.

The concentration calculation includes an additional correction factor. If the sample has been processed prior to use in dPCR, e.g. diluted, the processing and dilution steps should be included in the calculation in order to obtain the amount or concentration of a nucleic acid of interest in the sample analyzed. Therefore, in the exemplary dPCR plate, assuming the volume of one partition is 1 mL and the sample is diluted to one tenth concentration before dPCR, then the concentration before amplification is:

$N_c$ /(partition volume * number of filled partitions) = 17710/(11000 * 0.001L) = 1610 copies per liter.

Thus, before dilution there is 10*1610 = 16100 copies per liter.

The methods described herein are used to determine the amount or concentration of a nucleic acid in a sample using a dPCR analysis. A sample in the present context is a quantity of material that is suspected of containing one or more nucleic acids that are to be detected or measured and quantified. As used herein, the term includes, without limitation, a specimen (e.g., a biopsy or medical specimen), a cell or tissue cultures, blood, blood serum, blood plasma, needle aspirate, urine, semen, seminal fluid, seminal plasma, prostatic fluid, excreta, tears, saliva, sweat, biopsy, ascites, cerebrospinal fluid, pleural fluid, amniotic fluid, peritoneal fluid, interstitial fluid, sputum, milk, lymph, bronchial and other lavage samples, or tissue extract samples. The source of the sample may be solid tissue as from a fresh, frozen and/or preserved organ or tissue sample or biopsy or aspirate; or cells from any time in gestation or development of the subject. The sample may contain compounds that are not naturally intermixed with the source of the sample in nature such as preservatives, anticoagulants, buffers, fixatives, nutrients, antibiotics, or the like.

As detailed above, the sample contains a nucleic acid of interest, the amount or concentration of which is to be determined in the method of the present disclosure. A nucleic acid is a biopolymer essential for all known forms of life. Therefore, nucleic acids may be used as indicator for a particular organism, but also e.g. in case of mutations or naturally occurring variants, as indicator for a disease. The nucleic acid of interest may be selected from the group consisting of DNA, cDNA, RNA and a mixture thereof, or is any other type of nucleic acid. The nucleic acid may contain non nucleic acid components. It may be naturally occurring, chemically synthesized or biotechnologically engineered. Specifically, the nucleic acid is selected from the group consisting of DNA, cDNA, RNA and a mixture thereof.

The nucleic acid may be indicative of a microorganism (such as a pathogen) and may be useful in the diagnosis of a disease, such as an infection. Infections may be caused by bacteria, viruses, fungi, and parasites or other nucleic acid containing objects. The pathogen may be exogenous (acquired from environmental or animal sources or from other persons) or endogenous (from the normal flora). Samples may be selected on the basis of signs and symptoms, should be representative of the disease process, and should be collected before administration of antimicrobial agents. The amount of the nucleic acid in the unprocessed sample may be indicative of the severity of the disease.

Alternatively, the nucleic acid may be indicative of a genetic disorder. A genetic disorder is a genetic problem caused by one or more abnormalities in the genome, especially a condition that is present from birth (congenital). Most genetic disorders are quite rare and affect one person in every several thousands or millions. Genetic disorders may or may not be heritable, i.e., passed down from the parents' genes. In non-heritable genetic disorders, defects may be caused by new mutations or changes to the DNA. In such cases, the defect will only be heritable if it occurs in the germ line. The same disease, such as some forms of cancer, may be caused by an inherited genetic condition in some people, by new mutations in other people, and mainly by environmental causes in still other people. Evidently, the amount of nucleic acid with mutation may be indicative of the disease state.

In a specific embodiment, the sample is a biological fluid taken from a pregnant mammal that comprises a maternal source and a fetal source of nucleic acids (e.g., RNA or DNA). In this specific embodiment, chromosomal dosage resulting from fetal aneuploidy can be detected using nucleic acids from a maternal sample. In addition to empirical determination of the frequency of nucleic acids from a particular chromosome, the proportion of fetal nucleic acids in the maternal sample is also useful in determining the risk of fetal aneuploidy based on chromosome dosage, as it will impact the level of variation that is statistically significant in terms of the risk calculation. Utilizing such information in calculating the risk of an aneuploidy in one or more fetal chromosomes allows for a more accurate result that reflects the biological differences between samples. The proportion of fetal DNA in a maternal sample is used as a part of the risk calculation, as fetal proportion provides important information on the expected statistical presence of chromosomal dosage. Variation from the expected statistical presence may be indicative of fetal aneuploidy, an in particular a fetal trisomy or monosomy of a particular chromosome.

In the methods of the present disclosure, the amount or concentration of nucleic acids is determined. The amount of substance is a standards-defined quantity. The International System of Units (SI) defines the amount of substance to be proportional to the number of elementary entities present, with the inverse of the Avogadro constant as the proportionality constant (in units of mol). The SI unit for amount of substance is the mole. The mole is defined as the amount of substance that contains an equal number of elementary entities as there are atoms in 12 g of the isotope carbon-12. Therefore, the amount of substance of a sample is calculated as the sample mass divided by the molar mass of the substance. In the present context, the "amount" usually refers to the number of copies of the nucleic acid sequence of interest.

In dPCR, the partition may be a miniaturized chamber of a microarray or a nanoarray, a chamber of a microfluidic device, a microwell or a nanowell, on a chip, in a capillary, on a nucleic acid binding surface or on a bead, especially in a microarray or on a chip. The methods described herein are particularly suited for use with array-based systems, including but not limited to commercialized digital PCR platforms such as micro-well chip-based BioMark® dPCR from Fluidigm, and through hole-based QuantStudio12k flex dPCR and 3D dPCR from Life Technologies. The microfluidic-chip-based dPCR can have up to several hundred partitions per panel. The QuantStudio 12k dPCR performs digital PCR analysis on an OpenArray® plate which contains 64 partitions per subarray and 48 subarrays in total, equating to a total of 3072 partitions per array.

Typically, the accuracy and more importantly the precision of determination by dPCR may be improved by using a greater number of partitions. One may use approximately, 100 to 200, 200 to 300, 300 to 400, 700 or more partitions, which are used for determining the amount or concentration in question by PCR. In a particular embodiment, dPCR is carried out identically in at least 100 partitions, particularly at least 1,000 partitions, especially at least 5,000 partitions. In a specific embodiment, dPCR is carried out identically in at least 10,000 partitions, particularly at least 50,000 partitions, especially at least 100,000 partitions.

For example, dPCR is carried out identically in an array having between at least 100-100,000 partitions, e.g., between at least 1,000 - 100,000 reaction sites, or between at least 10,000 - 100,000 reaction sites.

The methods described herein are performed in a laboratory instrument or system configured to conduct a digital nucleic acid amplification reaction. As used herein, the term "nucleic acid amplification reaction" relates to a method or reaction used in molecular biology to amplify a single copy or a few copies of a target DNA segment (analyte) to a detectable amount of copies of the DNA segment involving repeated cycles of temperature-dependent reactions with a polymerase. Each cycle may comprise at least a denaturation phase (e.g. 95°C for 30 seconds), an annealing phase (e.g. 65°C for 30 seconds), and an extension phase (e.g. 72°C for 2 minutes).

The dPCR plate may be in thermal contact with a thermoelectric element for heating and/or cooling the sample holder to predefined temperatures of the different phases. Typically, a nucleic acid amplification reaction consists of 20-40 repeated cycles and the signal intensity of the light emitted from the reaction volumes in the dPCR plate is measured by the detector after the nucleic acid amplification reaction is completed. Based on the measured signal light intensity the presence of nucleic acid in the sample can be determined.

A laboratory instrument for conducting nucleic acid amplification reactions is well known in the art, and can include one or more of the following components (a representative laboratory instrument, 200, is illustrated schematically in Fig. 2):

i. a sample preparation module, 201, which can be a component of the laboratory instrument or a separate system, including a pipetting device for pipetting sample and/or reagents into a dPCR plate, 202, and partitioning a sample into one or more reaction volumes in the array, 203, in the dPCR plate;

ii. a dPCR plate support/handling module, 204, which transports the dPCR plate within the laboratory instrument from one module to another;

iii. a thermocycling module, 205, comprising a thermoelectric element for heating and/or cooling the dPCR plate during the amplification reaction;

iv. a detection module, 206, comprising a light source configured to emit light towards the dPCR plate (or a subsection thereof) and a light detector configured to measure a signal light intensity of light emitted from the dPCR plate (or a subsection thereof); and

v. a control device, 207, e.g., any physical or virtual processing device comprising a processor, 208, which is configured to control the laboratory instrument and components thereof in a way that sample analysis steps are conducted by the laboratory instrument.

The sample preparation module may be contained within the housing of the laboratory instrument or it may be a separate, stand-alone device that is not contained within the laboratory instrument housing. In the embodiment in which the sample preparation module is a separate device, a dPCR plate is prepared in the sample preparation module and the plate is then transported (automatically or manually) to the dPCR plate support/handling module, 204, within the laboratory instrument.

Optionally, the control device may receive information from a data management unit regarding which steps need to be performed with a certain sample. The processor of the control device may, for instance, be embodied as a programmable logic controller adapted to execute a computer-readable program provided with instructions to perform operations of the laboratory instrument. One operation is to conduct a method for detecting reaction volume deviations in a dPCR system, as described herein.

One or more of the components of the laboratory instrument described above are illustrated, e.g., in U.S Patent Application No. 20080160525; US Patent No. 10,564,102; US Patent Publication No. 20180045641A1; European Patent No. 3299471B1; US Patent Publication No. 20180147574A1; and US Patent Publication No. 20180087090A1.

In addition, the present disclosure contemplates a computer program product comprising instructions to cause the laboratory instrument described herein to execute the steps of the method to detect reaction volume deviations in a dPCR plate as described herein. Moreover, the disclosure also provides a computer-readable medium having stored thereon the computer program product comprising instructions to cause the laboratory instrument as described herein to execute the steps of the method to detect reaction volume deviations as described herein.

Embodiments of the subject matter and the operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer programs, i.e., one or more modules of computer program instructions, encoded on computer storage medium for execution by, or to control the operation of, data processing apparatus. A module may include logic that is executed by a processor(s). "Logic," as used herein, refers to any information having the form of instruction signals and/or data that may be applied to affect the operation of a processor. Software is an example of logic.

A computer storage medium can be, or can be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or can be included in, one or more separate physical components or media (e.g., multiple CDs, disks, or other storage devices). The operations described in this specification can be implemented as operations performed by a data processing apparatus on data stored on one or more computer-readable storage devices or received from other sources.

The term "programmed processor" encompasses all kinds of apparatus, devices, and machines for processing data, including by way of example a programmable microprocessor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus also can include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random-access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include any number of clients and servers. A client and server are generally remote from each other and typically interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

Unless defined otherwise, all technical and scientific terms and any acronyms used herein have the same meanings as commonly understood by one of ordinary skill in the art in the field of the disclosure. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1 -56081 -569-8).

The disclosure is not limited to the particular methodology, protocols, and reagents described herein because they may vary. Although any methods and materials similar or equivalent to those described herein can be used in the practice of the present disclosure, the particular methods, and materials are described

As used herein and in the appended claims, the singular forms "a," "an," and "the" include plural reference unless the context clearly dictates otherwise. Similarly, the words "comprise," "contain" and "encompass" are to be interpreted inclusively rather than exclusively. Similarly, the word "or" is intended to include "and" unless the context clearly indicates otherwise. The term "plurality" refers to two or more.

**Claims**

1. A method for detecting reaction volume deviations in a digital polymerase chain reaction (dPCR) assay, wherein the dPCR assay comprises quantifying an amount or concentration of nucleic acid of interest in an array of partitions, the method comprising:

   (a) combining optical signals across (x, y) coordinates within the array using a convolution with a kernel function, wherein each partition is assigned a convolution value, and applying a kernel function of a distance function comprising:

$$Dist(x_1, y_1, x_2, y_2) = \sqrt{(x_1 - x_2)^2 + (y_1 - y_2)^2}$$

$$ker(d) = \begin{cases} 2 & d = 0 \\ e^{-1*\sigma*d} & otherwise \end{cases} ;$$

   (b) identifying valid partitions and void partitions by comparing the convolution value of each partition to a threshold convolution value; and optionally,
   (c) subjecting data collected in step (b) to one or more additional steps comprising: clustering and morphological image processing operations.

2. The method of claim 1 further comprising subjecting data collected in step (b) to morphological image processing operations including dilation, erosion, and combinations thereof.

3. The method of any one of the preceding claims further comprising subjecting data collected in step (b) to clustering comprising valid and/or void trimming.

4. The method of any one of the preceding claims wherein a void partition has a convolution value below the threshold convolution value and a valid partition has a convolution value above the threshold convolution value.

5. The method of any one of the preceding claims wherein the array comprises a plurality of channels and step (a) further comprises determining which channel(s) of the plurality of channels to use in the method by a useChannel flag,

$$signalSum[i] = \sum_{useChannel[ch]==T} signalChannelch[i]/maxChannelch .$$

6. The method of any of the preceding claims, wherein z represents a set of (x, y) coordinates of a first partition, and the convolution of z is:

$$Conv[z] = \frac{\sum_{isValidPartition(z')\wedge Dist(z,z')<=radius} signalSum[z'] * ker(Dist(z, z'))}{\sum_{isValidPartition(z')\wedge Dist(z,z')<=radius} ker(Dist(z, z'))} .$$

7. The method of claim 6, wherein if

$$\{isValidPartition(z') \wedge Dist(z, z') <= radius\}$$

is an empty set, then an output for the empty set is set to a default value outside of the range of the convolution.

8. The method of any one of the preceding claims, wherein the convolution threshold is based on a set of convolution values within a selected reference region of the array.

9. The method of claim 8 wherein the selected reference region is selected from a vertical reference region, *i,* a horizontal reference region, *j,* and combinations thereof, wherein max x and max y are the maximum x and y coordinates of partitions in the vertical and/or horizontal reference region(s),

(a) the vertical reference region, i, is represented by

$$ref_i = \left\{ z \middle| (\frac{i}{5} \times maxy \leq z_y < \frac{i+1}{5} \times maxy \right\};$$

(b) the horizontal reference region, j, is represented by

$$ref_j = \left\{ z \middle| (11 \leq z_y < 0.5 \times maxy \wedge \frac{j}{3} \times maxx + \frac{maxx}{6} \leq z_x \leq \frac{j}{3} \times maxx + \frac{maxx}{2} \right\},$$

and for each vertical and/or horizontal reference region, the method further comprises calculating a mean of a valid convolution value of the vertical and/or horizontal reference region, and identifying as the selected reference region the vertical and/or horizontal reference region having a second highest mean convolution value.

10. The method of claim 9 further comprising calculating a median absolute deviation (MAD), expressed as (*median*(| *mean*(*ref*) - *ref*[*z*]|)) and excluding a default convolution value to yield a standard threshold:

$$voidThresh = mean(ref) - diffOff \times MAD(ref).$$

11. The method of claim 10, wherein the method further comprises using an alternative threshold if *mean(ref) > highConvolutionThreshold and MAD(ref) < low Variance Threshold,* wherein the alternative threshold is:

$$voidThresh = mean(ref) * thresholdAdjustmentFrac.$$

wherein *thresholdAdjustmentFrac* is a fraction of the mean used as the alternative threshold.

12. The method of any one of the preceding claims, wherein clustering comprises path connectedness including (a) grouping partitions in the array that are all pairwise connected to one another by a contiguous path, wherein the grouping is a cluster, (b) identifying one or more clusters having a size less than a void noise threshold value, and (c) designating a cluster identified in step (b) as valid.

13. The method of any one of the preceding claims, further comprising dilation to remove boundary voids.

14. The method of claim 13, wherein for a valid partition with coordinate z, the valid partition is designated as a void partition if there exists a void partition z' with $|z_x - z'_x| + |z_y - z'y| \leq$ *cleanupRadius.*

15. The method of any one of the preceding claims, wherein clustering comprises path connectedness including (a) grouping partitions in the array that are all pairwise connected to one another by a continguous path, wherein the grouping is a cluster, (b) identifying one or more clusters having a size less than a valid noise threshold value, and (c) designating a cluster identified in step (b) as void.

16. The method of any one of the preceding claims further comprising flagging partitions identified as void.

17. A computer program product comprising instructions to cause a laboratory instrument to execute the steps of the method according to any one of claims 1-16.

**Patentansprüche**

1. Verfahren zum Detektieren von Reaktionsvolumenabweichungen in einem digitalen Polymerasekettenreaktions-(dPCR)-Assay, wobei der dPCR-Assay das Quantifizieren einer Menge oder Konzentration von interessierender Nukleinsäure in einem Array von Partitionen umfasst, wobei das Verfahren Folgendes umfasst:

(a) Kombinieren von optischen Signalen über (x, y)-Koordinaten innerhalb des Arrays unter Verwendung einer Faltung mit einer Kernelfunktion, wobei jeder Partition ein Faltungswert zugewiesen wird, und Anwenden einer Kernelfunktion einer Abstandsfunktion, die Folgendes umfasst:

$$Dist(x_1, y_1, x_2, y_2) = \sqrt{(x_1 - x_2)^2 + (y_1 - y_2)^2}$$

$$ker(d) = \begin{cases} 2 & d = 0 \\ e^{-1*\sigma*d} & anderenfalls \end{cases};$$

(b) Identifizieren gültiger Partitionen und leerer Partitionen durch Vergleichen des Faltungswerts jeder Partition mit einem Faltungsschwellenwert; und gegebenenfalls

(c) Unterziehen von in Schritt (b) gesammelten Daten einem oder mehreren zusätzlichen Schritten, die Folgendes umfassen: Clustering und morphologische Bildverarbeitungsoperationen.

2. Verfahren nach Anspruch 1, ferner umfassend das Unterziehen von in Schritt (b) gesammelten Daten morphologischen Bildverarbeitungsoperationen, einschließlich Dilatation, Erosion und Kombinationen davon.

3. Verfahren nach einem der vorhergehenden Ansprüche, ferner umfassend das Unterziehen von in Schritt (b) gesammelten Daten einem Clustering, umfassend Trimmen gültiger und/oder ungültiger Daten.

4. Verfahren nach einem der vorhergehenden Ansprüche, wobei eine leere Partition einen Faltungswert unter dem Faltungsschwellenwert aufweist und eine gültige Partition einen Faltungswert über dem Faltungsschwellenwert aufweist.

5. Verfahren nach einem der vorhergehenden Ansprüche, wobei das Array eine Vielzahl von Kanälen umfasst und Schritt (a) ferner das Bestimmen, welcher Kanal bzw. welche Kanäle der Vielzahl von Kanälen in dem Verfahren zu verwenden sind, durch ein useChannel-Flag umfasst.

$$signalSum[i] = \sum_{useChannel[ch]==T} signalChannelch[i]/maxChannelch$$

.

6. Verfahren nach einem der vorhergehenden Ansprüche, wobei z einen Satz von (x, y)-Koordinaten einer ersten Partition darstellt und die Faltung von z wie folgt ist:

$$Conv[z] = \frac{\sum_{isValidPartition(z') \wedge Dist(z,z') <= radius} signalSum[z'] * ker(Dist(z,z'))}{\sum_{isValidPartition(z') \wedge Dist(z,z') <= radius} ker(Dist(z,z'))}$$

.

7. Verfahren nach Anspruch 6, wobei, wenn

$$\{isValidPartition(z') \wedge Dist(z,z') <= radius\}$$

ein leerer Satz ist, eine Ausgabe für den leeren Satz auf einen Standardwert außerhalb des Bereichs der Faltung eingestellt wird.

8. Verfahren nach einem der vorhergehenden Ansprüche, wobei der Faltungsschwellenwert auf einem Satz von Faltungswerten innerhalb einer ausgewählten Referenzregion des Arrays basiert.

9. Verfahren nach Anspruch 8, wobei die ausgewählte Referenzregion aus einer vertikalen Referenzregion, *i,* einer horizontalen Referenzregion, j und Kombinationen davon ausgewählt ist, wobei max x und max y die maximalen x- und y-Koordinaten von Partitionen in der vertikalen und/oder horizontalen Referenzregion bzw. den vertikalen und/oder horizontalen Referenzregionen sind,

(a) die vertikale Referenzregion i durch Folgendes dargestellt wird:

$$ref_i = \left\{ z \middle| (\frac{i}{5} \times maxy \ \leq\ z_y < \frac{i+1}{5} \times maxy) \right\};$$

(b) die horizontale Referenzregion *j* durch Folgendes dargestellt wird:

$$ref_j = \left\{ z \middle| (11 \leq z_y < 0.5 \ \times maxy \ \wedge \frac{j}{3} \times maxx + \frac{maxx}{6} \leq z_x \leq \frac{j}{3} \times maxx + \frac{maxx}{2} \right\},$$

und für jede vertikale und/oder horizontale Referenzregion das Verfahren ferner das Berechnen eines Mittelwerts eines gültigen Faltungswerts der vertikalen und/oder horizontalen Referenzregion und das Identifizieren der vertikalen und/oder horizontalen Referenzregion mit einem zweithöchsten mittleren Faltungswert als die ausgewählte Referenzregion umfasst.

10. Verfahren nach Anspruch 9, das ferner das Berechnen einer medianen absoluten Abweichung (MAD), ausgedrückt als (*median*(|*mean*(*ref*) - *ref*[*z*]|)) und das Ausschließen eines Standardfaltungswerts umfasst, um einen Standardschwellenwert zu erhalten:

$$voidThresh = mean(ref) - diffOff \times MAD(ref).$$

11. Verfahren nach Anspruch 10, wobei das Verfahren ferner das Verwenden eines alternativen Schwellenwerts umfasst, wenn *mean*(*rej*) > *highConvolutionThreshold* und *MAD*(*rej*) < *lowVarianceThreshold,* wobei der alternative Schwellenwert Folgendes ist:

$$voidThresh = mean(ref) * thresholdAdjustmentFrac.$$

wobei *thresholdAdjustmentFrac* ein Bruchteil des Mittelwerts ist, der als der alternative Schwellenwert verwendet wird.

12. Verfahren nach einem der vorhergehenden Ansprüche, wobei das Clustering eine Pfadverbundenheit umfasst, einschließlich (a) Gruppieren von Partitionen in dem Array, die alle paarweise durch einen zusammenhängenden Pfad miteinander verbunden sind, wobei die Gruppierung ein Cluster ist, (b) Identifizieren eines oder mehrerer Cluster mit einer Größe, die kleiner als ein Leerrausch-Schwellenwert ist, und (c) Bezeichnen eines in Schritt (b) identifizierten Clusters als gültig.

13. Verfahren nach einem der vorhergehenden Ansprüche, ferner umfassend Dilatation, um Grenz-Leerräume zu entfernen.

14. Verfahren nach Anspruch 13, wobei für eine gültige Partition mit Koordinate z die gültige Partition als leere Partition bezeichnet wird, wenn eine leere Partition z' mit $|z_x - z'_x| + |z_y - z'_y| \leq cleanupRadius$ existiert.

15. Verfahren nach einem der vorhergehenden Ansprüche, wobei das Clustering eine Pfadverbundenheit umfasst, einschließlich (a) Gruppieren von Partitionen in dem Array, die alle paarweise durch einen zusammenhängenden Pfad miteinander verbunden sind, wobei die Gruppierung ein Cluster ist, (b) Identifizieren eines oder mehrerer Cluster mit einer Größe, die kleiner als ein gültiger Rauschschwellenwert ist, und (c) Bezeichnen eines in Schritt (b) identifizierten Clusters als leer.

16. Verfahren nach einem der vorhergehenden Ansprüche, ferner umfassend das Flagging von als leer identifizierten Partitionen.

17. Computerprogrammprodukt, umfassend Anweisungen, um zu bewirken, dass ein Laborinstrument die Schritte des Verfahrens nach einem der Ansprüche 1-16 ausführt.

## Revendications

1. Procédé de détection d'écarts de volume de réaction dans un dosage par réaction en chaîne par polymérase numérique (dPCR), dans lequel le dosage par dPCR comprend la quantification d'une quantité ou d'une concentration d'acide nucléique d'intérêt dans un réseau de partitions, le procédé comprenant :

   (a) la combinaison de signaux optiques sur des coordonnées (x, y) au sein du réseau en utilisant une convolution avec une fonction noyau, dans lequel chaque partition se voit attribuer une valeur de convolution, et l'application d'une fonction noyau d'une fonction de distance comprenant :

   $$Dist(x_1, y_1, x_2, y_2) = \sqrt{(x_1 - x_2)^2 + (y_1 - y_2)^2}$$

   $$ker(d) = \begin{cases} 2 & d = 0 \\ e^{-1*\sigma*d} & sinon \end{cases};$$

   (b) l'identification de partitions valides et de partitions vides en comparant la valeur de convolution de chaque partition à une valeur de convolution seuil ; et éventuellement,
   (c) la soumission des données recueillies à l'étape (b) à une ou plusieurs étapes supplémentaires comprenant : un regroupement et des opérations de traitement d'image morphologiques.

2. Procédé selon la revendication 1 comprenant en outre la soumission des données recueillies à l'étape (b) à des opérations de traitement d'image morphologiques comprenant la dilatation, l'érosion et des combinaisons de celles-ci.

3. Procédé selon l'une quelconque des revendications précédentes comprenant en outre la soumission des données recueillies à l'étape (b) au regroupement comprenant un élagage des partitions valides et/ou vides.

4. Procédé selon l'une quelconque des revendications précédentes dans lequel une partition vide a une valeur de convolution inférieure à la valeur de convolution seuil et une partition valide a une valeur de convolution supérieure à la valeur de convolution seuil.

5. Procédé selon l'une quelconque des revendications précédentes dans lequel le réseau comprend une pluralité de canaux et l'étape (a) comprend en outre la détermination du ou des canaux de la pluralité de canaux à utiliser dans le procédé au moyen d'un indicateur useChannel,

$$signalSum[i] = \sum_{useChannel[ch]==T} signalChannelch[i]/maxChannelch$$

6. Procédé selon l'une quelconque des revendications précédentes, dans lequel z représente un ensemble de coordonnées (x,y) d'une première partition, et la convolution de z est :

$$Conv[z] = \frac{\sum_{isValidPartition(z') \wedge Dist(z,z')<=radius} signalSum[z'] * ker(Dist(z,z'))}{\sum_{isValidPartition(z') \wedge Dist(z,z')<=radius} ker(Dist(z,z'))}$$

7. Procédé selon la revendication 6, dans lequel si

$$\{isValidPartition(z') \wedge Dist(z,z') <= radius\}$$

est un ensemble vide, alors une sortie pour l'ensemble vide est définie à une valeur par défaut hors de la plage de la convolution.

8. Procédé selon l'une quelconque des revendications précédentes, dans lequel le seuil de convolution est basé sur un ensemble de valeurs de convolution au sein d'une région de référence choisie du réseau.

9. Procédé selon la revendication 8 dans lequel la région de référence choisie est choisie parmi une région de référence verticale, $i$, une région de référence horizontale, $j$, et des combinaisons de celles-ci, dans lequel max x et max y sont les coordonnées x et y maximales des partitions dans la ou les régions de référence verticale et/ou horizontale,

(a) la région de référence verticale, i, est représentée par

$$ref_i = \left\{ z \middle| (\frac{i}{5} \times maxy \ \leq z_y < \frac{i+1}{5} \times maxy \right\};$$

(b) la région de référence horizontale, j, est représentée par

$$ref_j = \left\{ z \middle| (11 \leq z_y < 0.5 \ \times maxy \ \wedge \frac{j}{3} \times maxx + \frac{maxx}{6} \leq z_x \leq \frac{j}{3} \times maxx + \frac{maxx}{2} \right\},$$

et pour chaque région de référence verticale et/ou horizontale, le procédé comprend en outre le calcul d'une moyenne d'une valeur de convolution valide de la région de référence verticale et/ou horizontale, et l'identification en tant que région de référence choisie de la région de référence verticale et/ou horizontale ayant une deuxième valeur de convolution moyenne la plus élevée.

10. Procédé selon la revendication 9 comprenant en outre le calcul d'un écart absolu médian (MAD), exprimé en tant que ($median$(|$mean$($rej$) - $ref$[$z$]|)) et excluant une valeur de convolution par défaut pour obtenir un seuil standard :

$$voidThresh = mean(ref) - diffOff \times MAD(ref).$$

11. Procédé selon la revendication 10, dans lequel le procédé comprend en outre l'utilisation d'un seuil alternatif si $mean(ref) > highConvolutionThreshold$ et $MAD(ref) < lowVarianceThreshold,$ dans lequel le seuil alternatif est :

$$voidThresh = mean(ref) * thresholdAdjustmentFrac.$$

dans lequel $thresholdAdjustmentFrac$ est une fraction de la moyenne utilisée en tant que seuil alternatif.

12. Procédé selon l'une quelconque des revendications précédentes, dans lequel le regroupement comprend une connectivité par chemin comprenant (a) le groupement des partitions dans le réseau qui sont toutes connectées par paires les unes aux autres par un chemin contigu, dans lequel le groupement est un cluster, (b) l'identification d'un ou de plusieurs clusters ayant une taille inférieure à une valeur seuil de bruit de vide, et (c) la désignation d'un cluster identifié à l'étape (b) comme valide.

13. Procédé selon l'une quelconque des revendications précédentes comprenant en outre une dilatation pour retirer les vides aux frontières.

14. Procédé selon la revendication 13, dans lequel pour une partition valide avec la coordonnée z, la partition valide est désignée comme étant une partition vide s'il existe une partition vide z' avec |$z_x$ - $z'_x$| + |$z_y$ - $z'_y$| $\leq$ $cleanupRadius.$

15. Procédé selon l'une quelconque des revendications précédentes, dans lequel le regroupement comprend une connectivité par chemin comprenant (a) le groupement des partitions dans le réseau qui sont toutes connectées par paires les unes aux autres par un chemin contigu, dans lequel le groupement est un cluster, (b) l'identification d'un ou plusieurs clusters ayant une taille inférieure à une valeur seuil de bruit valide, et (c) la désignation d'un cluster identifié à l'étape (b) comme vide.

16. Procédé selon l'une quelconque des revendications précédentes comprenant en outre le marquage des partitions identifiées comme vides.

17. Produit de programme informatique comprenant des instructions pour amener un instrument de laboratoire à exécuter les étapes du procédé selon l'une quelconque des revendications 1 à 16.

Fig. 1A

Feature Calculation (101)

Identification of valid/void partitions (102)

Optional clean-up step (103)

Fig. 1B

```
┌─────────────────────┐      ┌─────────────────────┐      ┌─────────────────────┐
│ Sample prep: partition │      │ Subject dPCR plate to │      │ Collect signal data from │
│ sample in dPCR plate  │ ───> │ amplification (105)   │ ───> │ dPCR plate (106)      │
│ (104)                │      │                      │      │                      │
└─────────────────────┘      └─────────────────────┘      └─────────────────────┘
                                                                      │
                    ┌─────────────────────────────────────────────────┘
                    ▼
┌─────────────────────┐      ┌─────────────────────┐      ┌─────────────────────┐
│ Feature compution:   │      │ Set threshold for    │      │ Compare partition    │
│ combine signals across│ ───> │ convolution values   │ ───> │ features to threshold │
│ channels & convolve  │      │ (108)                │      │ and determine        │
│ with a kernel (107)  │      │                      │      │ valid/void (109)     │
└─────────────────────┘      └─────────────────────┘      └─────────────────────┘
                                                                      │
                    ┌─────────────────────────────────────────────────┘
                    ▼
┌─────────────────────┐      ┌─────────────────────┐      ┌─────────────────────┐
│ Void trimming        │      │ Dilation (optional) (111) │ ───> │ Valid trimming       │
│ (optional) (110)     │ ───> │                      │      │ (optional) (112)     │
└─────────────────────┘      └─────────────────────┘      └─────────────────────┘
```

EP 4 143 337 B1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130302792 A1
- US 2018230515 A
- US 20080160525 A
- US 10564102 B
- US 20180045641 A1
- EP 3299471 B1
- US 20180147574 A1
- US 20180087090 A1

**Non-patent literature cited in the description**

- **VOGELSTEIN et al.** *Proc. Natl. Acad. Sci. USA*, August 1999, vol. 96, 9236-9241
- **MCCAUGHAN et al.** *J. Pathol.*, 2010, vol. 220, 297-306
- **MAO et al.** *Am. J. Transl. Res.*, 2019, vol. 11 (12), 7209-7222
- The Encyclopedia of Molecular Biology. Blackwell Science Ltd, 1994
- Molecular Biology and Biotechnology: a Comprehensive Desk Reference. VCH Publishers, Inc, 1995